# EUROPEAN PATENT SPECIFICATION

(11) **EP 2 570 441 B1**
(45) Date of publication and mention of the grant of the patent: **01.04.2015**
(21) Application number: 12185044.0
(22) Date of filing: 14.10.2009
(51) Int. Cl.: C08G 18/40, A61K 31/785, A61L 24/00, A61L 24/04, A61L 24/08, A61L 27/00, A61L 31/00, A61L 27/14, A61L 27/58

(54) **Bioabsorbable surgical composition**
Bioabsorbierbare chirurgische Zusammensetzung
Composition chirurgicale bioabsorbable

(30) Priority: 15.10.2008 US 105482 P; 02.10.2009 US 572358
(43) Date of publication of application: 20.03.2013
(62) Divisional of application: 09252410.7
(73) Proprietor: Covidien LP, Mansfield, MA 02048 (US)
(72) Inventor: Stopek, Joshua, Yalesville, CT Connecticut 06492 (US); Hadba, Ahmad, Middlefield, CT Connecticut 06455 (US)
(74) Representative: Tanner, James Percival

(56) References cited:
- WO-A1-2006/002506
- WO-A2-03/059296

## Description

### TECHNICAL FIELD

The present disclosure relates to compositions suitable for application in situ, including for use as tissue adhesives and/or tissue sealants.

### DESCRIPTION OF THE RELATED ART

Matrix metalloproteinases (MMPs) are neutral zinc-dependent endopeptidases with substrate specificity for most extracellular matrix molecules, including collagens, gelatins, fibronectin, laminin and proteoglycan. They depend upon zinc for their catalytic activity.

Most cells do not express MMPs in vivo. Instead, growth factors, hormones, inflammatory cytokines, cell-matrix interactions and cellular transformation regulate their expression. Although the secretory granules of neutrophils and eosinophils are known to store some MMPs, most cell types normally synthesize very low quantities of MMPs.

MMPs share some common structural characteristics that include a signal sequence, an amino-terminal pro-peptide domain, a catalytic zinc binding domain, a proline-rich hinge region, and a carboxy-terminal hemopexin-like domain.

Extracellular matrix degradation is a normal event in the physiological remodeling associated with morphogenesis, reproduction, and in growth and maintenance processes such as cell migration, angiogenesis, and tissue regeneration. During inflammation and in several disease situations, however, excess MMPs may degrade the surrounding proteinaceous matrix, which may result in the destruction or weakening of connective tissue, unregulated cell migration/invasion, and/or tissue fibrosis. For example, connective tissue weakening or destruction may result in diseases such as rheumatoid arthritis, osteoarthritis, chronic periodontis, and arterial and cardiac aneurysm. Accordingly, MMP inhibitors have been used to treat osteoporosis, osteoarthritis, human chronic periodontal disease and various types of aneurysms.

In recent years there has developed increased interest in replacing or augmenting sutures with adhesive bonds. Studies in this area, however, have revealed that in order for surgical adhesives to be accepted by surgeons, they must possess a number of properties. They must exhibit high initial tack and an ability to bond rapidly to living tissue; the strength of the bond should be sufficiently high to cause tissue failure before bond failure; the adhesive should form a bridge, typically a permeable flexible bridge; and the adhesive bridge and/or its metabolic products should not cause local histotoxic or carcinogenic effects.

Several materials useful as tissue adhesives or tissue sealants are currently available. One type of adhesive that is currently available is a cyanoacrylate adhesive. One, disadvantage with cyanoacrylate adhesives is that they can have a high flexural modulus which can limit their usefulness.

Another type of tissue sealant that is currently available utilizes components derived from bovine and/or human sources. For example, fibrin sealants are available. However, as with any natural material, variability in the material is frequently observed and, because the sealant is derived from natural proteins, there may be viral transmission concerns.

Document V110 03/059296 A2 relates to surgical sealants for tissue healing (page 37-39). A composition comprising a biodegradable polymer (based on DL-Lactide), methoxy polyethylenglycol (MePEG 2000) and a hydroxamate associated with the polymer by a covalent linkage is described in the examples 9, 10, as well as in claims 8, 19, 20-21 and 24.

Document WO 2006/002506 A1 discloses therapeutic polymers used as wound care products. Claims 1, 10, 12, 14, 22, 28 and 34 relate to a hydroxamate-containing polymer comprising polyethylene glycol and an anhydride which is bioabsorbable.

It would be desirable to provide a biological adhesive that is highly consistent in its properties, without the concern of viral transmission, and which may promote wound healing. Such a composition should be flexible and biocompatible and should be suitable for use as an adhesive or sealant.

### SUMMARY

The present invention is defined in independent claim 1 and certain optional features thereof are defined in the dependent claims.

The present disclosure provides bioabsorbable compositions suitable for use in vivo, as well as methods for their use. In embodiments, a bioabsorbable macromer composition of the present disclosure may be of the formula:

R₁-[A]ᵥ-R₂ (III)

and/or

R₂-[A]ᵥ-R₁-[A]ᵥ-R₂ (IV)

wherein R₁ is a hydroxamate segment, R₂ is a polymer segment such as polysaccharides and polyols, A is a bioabsorbable group, and v is a number from 1 to 20.

In other embodiments, a bioabsorbable macromer of the present disclosure may be of the formula:

R₁-[A]ᵥ-R₂-R₃ (V)

and/or

R₃-R₂-[A]ᵥ-R₁-[A]ᵥ-R₂-R₃ (VI)

wherein R₁ is a hydroxamate segment, R₂ is a polymer such as polysaccharides and polyols, R₃ is a functional group such as isocyanates, succinimides, aldehydes, and combinations thereof, A is a bioabsorbable group, and v is a number from 1 to 20.

In yet other embodiments, a bioabsorbable macromer composition of the present disclosure may include a polymeric component of the formula:

R₃-R₂-[A]ᵥ-R₁-[A]ᵥ-R₂-R₃ (VI)

wherein R₁ is a hydroxamate segment, R₂ is a polymer such as polysaccharides and polyols, R₃ is a functional group such as isocyanates, succinimides, aldehydes, and combinations thereof, A is a bioabsorbable group, and v is a number from 1 to 20; and a second component possessing at least one group reactive with the functional component of the polymeric component.

In embodiments, the hydroxamate segment of the compositions of the present disclosure may include at least one hydroxamate of the formula : 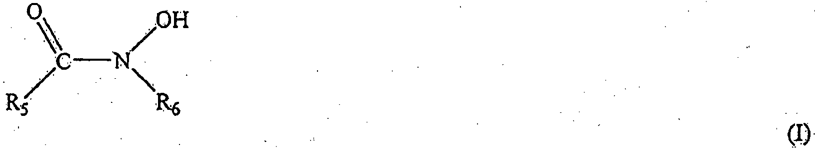 wherein R₅ can be vinyl groups, hydroxyl alkyl acrylate groups, hydoxy alkyl methacrylate groups, alkyl groups, alkoxy groups, alkenyl groups, acrylamides, methacrylamides, polymers, and combinations thereof, and R₆ can be hydrogen, alkyl groups, alkoxy groups, alkenyl groups, and combinations thereof.

The present disclosure also provides methods for using the compositions of the present disclosure. Such methods may include, but are not limited to, methods for closing a wound with the compositions of the present disclosure, methods for filling a void in animal tissue with a composition of the present disclosure, and methods for adhering a medical device to a surface of animal tissue with a composition of the present disclosure.

The invention may be described by reference to the following numbered paragraphs:-

A bioabsorbable macromer composition according to the invention for use in closing a wound wherein the method comprises the step of applying the composition to the wound; and allowing the bioabsorbable macromer composition to set thereby closing the wound.

The method according to the invention wherein the wound is a surgical incision.

A bioabsorbable macromer composition according to the invention for use in filling a void in animal tissue wherein the method comprises the steps of applying the composition to the void; and allowing the bioabsorbable macromer composition to set thereby filling the void.

A method for adhering a medical device to a surface of animal tissue comprising the steps of:
applying the bioabsorbable macromer composition according to the invention to the device, the surface or both;
bringing the device, bioabsorbable macromer composition and surface into contact with each other; and
allowing the bioabsorbable macromer composition to set thereby adhering the device and surface to each other.

The method paragraph [0020], wherein said medical device is an implant.

### DETAILED DESCRIPTION

The present disclosure relates to a macromer composition for use as a tissue adhesive or sealant, which is biocompatible, non-immunogenic and biodegradable. The bioabsorbable macromer composition can be applied to living tissue and/or flesh of animals, including humans. The bioabsorbable macromer composition can be employed to adhere tissue edges, to seal air/fluid leaks in tissues, to adhere medical devices, i.e., implants, and for tissue augmentation such as sealing or filling voids or defects in tissue. The composition may also be utilized as a tissue protective coating, an anti-adhesive coating, a drug delivery vehicle, and the like.

While certain distinctions may be drawn between the usage of the terms "flesh" and "tissue" within the scientific community, the terms are used interchangeably herein as referring to a general substrate upon which those skilled in the art would understand the present bioabsorbable macromer composition to be utilized within the medical field for the treatment of patients. As used herein, "tissue" may include, but is not limited to, skin, bone, neuron, axon, cartilage, blood vessel, cornea, muscle, fascia, brain, prostate, breast, endometrium, lung, pancreas, small intestine, blood, liver, testes, ovaries, cervix, colon, stomach, esophagus, spleen, lymph node, bone marrow, kidney, peripheral blood, embryonic and/or ascite tissue.

The composition of the present disclosure includes a polymeric component possessing a hydroxamate segment in combination with a polymer segment, which may be a polymer, oligomer, and/or macromer. As used herein, a "polymer segment" may include a polymer, oligomer, and/or macromer. As used herein, an oligomer may include repeating monomeric units of from about 4 repeat units to about 50 repeat units, in embodiments from about 5 repeat units to about 20 repeat units. A macromer of the present disclosure is of a longer length, of from about 50 repeat units to about 500 repeat units, in embodiments from about 75 repeat units to about 200 repeat units. A polymer of the present disclosure is of a longer length of from about 500 repeat units to about 20,000 repeat units, in embodiments from about 750 repeat units to about 10,000 repeat units.

The hydroxamate segment, the polymer segment, or both, may be conjugated to biomolecules including proteins, peptides, polysaccharides, synthetic polymers or oligomers, alkylene oxides, polymer drugs, composites including the foregoing, combinations thereof, and the like, utilizing methods within the purview of those skilled in the art.

Suitable hydroxamate segments which may be utilized in forming compositions of the present disclosure include, but are not limited to, segments possessing a hydroxamate group of the following formula (I): 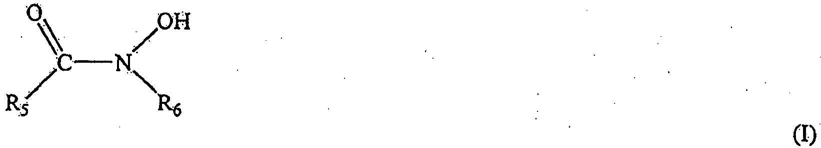 wherein R₅ may include vinyl groups, acrylate groups including hydroxy alkyl acrylates, methacrylate groups including hydroxy alkyl methacrylates, other alkyl groups, alkoxy groups, alkenyl groups, acrylamide groups, methacrylamide groups, polymers, and combinations thereof, and R₆ may be hydrogen, alkyl groups, alkoxy groups, alkenyl groups, or combinations thereof.

As used herein, "alkyl", used either alone or in compound words such as "haloalkyl" or "alkylthio", includes straight chain or branched C₁₋₁₂ alkyl groups. Examples include methyl, ethyl, propyl, isopropyl, and the like.

As used herein, "alkoxy" includes straight chain or branched alkoxy, in embodiments C₁₋₁₂ alkoxy such as methoxy, ethoxy, n-propoxy, isopropoxy and butoxy isomers.

As used herein, "alkenyl" includes groups formed from straight chain, branched or mono- or polycyclic alkenes including ethylenically mono- or poly-unsaturated alkyl or cycloalkyl groups as previously defined, in embodiments C₂₋₁₂ alkenyl. Examples of alkenyl include vinyl; allyl; 1-methylvinyl; butenyl; iso-butenyl; 3-methyl-2-butenyl; 1-pentenyl; cyclopentenyl; 1-methyl-cyclopentenyl; 1-hexenyl, 3-hexenyl; cyclohexenyl; 1-heptenyl; 3-heptenyl; 1-octenyl; cyclooctenyl; 1-nonenyl, 2-nonenyl; 3-nonenyl; 1-decenyl; 3-decenyl; 1,3-butadienyl; 1-4,pentadienyl; 1,3-cyclopentadienyl; 1,3-hexadienyl; 1,4-hexadienyl; 1,3-cyclohexadienyl; 1,4-cyclohexadienyl; 1,3-cycloheptadienyl; 1,3,5-cycloheptatrienyl; or 1,3,5,7-cyclooctatetraenyl.

Methods for forming these hydroxamate functional compositions are within the purview of those skilled in the art. For example, in embodiments, a hydroxamate functional polymer may be produced by the surface modification of cross-linked polymethacrylic acid (PMAA)-co-methyl methacrylate (MAA) beads, thus producing a hydroxamate functional polymer, i.e., PMAA-MMA-hydroxamate, which may be utilized as the hydroxamate segment.

In other embodiments, polymerizable hydroxamate monomers may be synthesized which may be combined with the polymer segment to produce the polymeric component of the present disclosure. The hydroxamate monomer, which may be encompassed by formula I above, may have an R₅ including CH₂=C-CH₃, and R₆ may be hydrogen. In other embodiments, the hydroxamate monomer may be utilized to synthesize a hydroxamate homopolymer, or may be copolymerized with any other suitable comonomers to produce copolymers which, in turn, may be combined with the polymer segment to produce the polymeric component of the present disclosure.

Hydroxamate homopolymers synthesized from the above hydroxamate monomer can also be grafted onto any derivatizable polymer. The resulting hydroxamate functional unit, whether a monomer, homopolymer, or copolymer, may then be combined with the polymer segment to produce the polymeric component of the present disclosure.

It should, of course be understood that two or more hydroxamates may be utilized in forming the polymeric component of the present disclosure.

As noted above, the polymeric component of the present disclosure also includes a polymer segment. In embodiments, a suitable polymer segment may include anionic polysaccharides such as carboxymethyl cellulose (CMC), alginate, chitosan and hyaluronic acid; proteins including gelatin, collagen and albumin; polypeptides including poly(glutamic) acid, poly(lysine), and copolymers of multiple amino acids; polyols including polyalkylene oxides, polyvinyl alcohols, combinations thereof, and the like; and copolymers of polyalkylene oxides such as polyethylene glycol with degradable polymers prepared from L-lactide, DL-lactide, glycolide, s-caprolactone, p-dioxanone, trimethylene carbonate, combinations thereof, and the like. Combinations of any of the foregoing may be utilized in embodiments.

In other embodiments, ultraviolet polymerizable or curable oligomers, macromers, or polymers may be utilized as the polymer segment, including acrylates such as polyethylene glycol acrylate, polyethylene glycol diacrylate, polyethylene glycol fumarate, polycaprolactone fumarate, polyglycolide fumarate, polylactide fumarate, copolymers thereof, combinations thereof, and the like.

In some embodiments, the polymer segment can be a polysaccharide such as sorbitol, mannitol, sucrose, dextran, cyclodextrin, combinations thereof, and the like.

In other embodiments, the polymer segment can be a polyol such as a polyalkylene oxide including polyethylene oxide ("PEO"), polypropylene oxide ("PPO"), polyethylene glycol ("PEG"), polypropylene glycol ("PPG"), a polyethylene glycol with lactide linkages, polyethylene glycol-adipate, co-polyethylene oxide block or random copolymers, polyethylene glycol-polypropylene glycol copolymers, including poloxamers such as polyethylene oxide (PEO) copolymers with polypropylene oxide (PPO), for example the triblock PEO - PPO copolymers commercially available as PLURONICS^{®} from BASF Corporation (Mt. Olive, NJ), combinations thereof, and the like.

In embodiments a polyalkylene oxide may be utilized as the polymer, such as a polyethylene oxide, for example a polyethylene glycol ("PEG"). As used herein, polyethylene glycol generally refers to a polymer with a molecular weight of less than about 50,000, while polyethylene oxide is used for higher molecular weights. PEGs provide excellent water retention, flexibility and viscosity in the biocompatible synthetic macromer composition.

In embodiments, a PEG may be utilized as the polymer segment having a molecular weight of from about 100 to about 20,000, in embodiments from about 500 to about 10,000, in other embodiments from about 1,000 to about 5,000.

In embodiments, the composition of the present disclosure, including the hydroxamate segment and polymer segment described above, may include bioabsorbable groups. Bioabsorbable groups are within the purview of those skilled in the art and can include those which undergo hydrolytic degradation. Suitable bioabsorbable groups include hydrolytically labile α-hydroxy acids such as lactic acid and glycolic acid, glycolide, lactide, lactones including ε-caprolactone, carbonates such as trimethylene carbonate, ester ethers such as dioxanones including 1,4-dioxane-2-one and 1,3-dioxane-2-one, diacids including succinnic acid, adipic acid, sebacic acid, malonic acid, glutaric acid, azelaic acid, phosphoesters such as ethyl dichlorophosphate, anhydrides such as sebacic acid anhydride and azelaic acid anhydride, etc., and combinations thereof.

Methods for introducing these bioabsorbable groups into the hydroxamate segment and/or polymer segment are within the purview of those skilled in the art. For example, a bioabsorbable group may be incorporated by first reacting the hydroxamate segment with a polyhydric alcohol such as D-sorbitol, D-mannitol, tris(hydroxymethyl)aminomethane (also known as 2-amino-2-(hydroxymethyl)-1,3-propanediol), diethylene glycol, threitol, pentaerythritol, enterodiol, cyclodextrins, etc. to form a hydroxamate segment having multiple hydroxy groups, i.e.,

R₁-(OH)ₙ (II)

where R₁ is the hydroxamate segment, and n is a number from about 1 to about 20. In other embodiments, the hydroxamate segment may be of formula I above, which already possesses a hydroxyl group.

The hydroxamate segment having multiple hydroxy groups may then, in turn, be reacted with a hydroxy acid such as lactic acid, glycolic acid, or other bioabsorbable groups as described above, including lactones, to form a hydroxamate segment having multiple bioabsorbable/hydroxy groups.

The polymer segment described above may then be reacted with the bioabsorbable group to form a polymeric composition including the hydroxamate segment, the bioabsorbable group, and the polymer segment.

Methods for reacting the polymer segment with the hydroxamate segment, optionally possessing bioabsorbable groups, are within the purview of those skilled in the art. For example, in some embodiments, a hydroxamate segment having hydroxyl functionality may be reacted with a diacid such as malonic acid, succinic acid, glutaric acid, adipic acid, sebacic acid, azelaic acid, combinations thereof, and the like, to produce a component having carboxylic acid functionality. The component having carboxylic functionality may then be reacted with an amine such as 4-(p-azidosalicylamido-butylamine) (ASBA) in combination with a carbodiimide such as EDC (1-ethyl -3(3-dimethyl-amino propyl)-carbodiimide hydrochloride). An overview of the reaction is as follows: 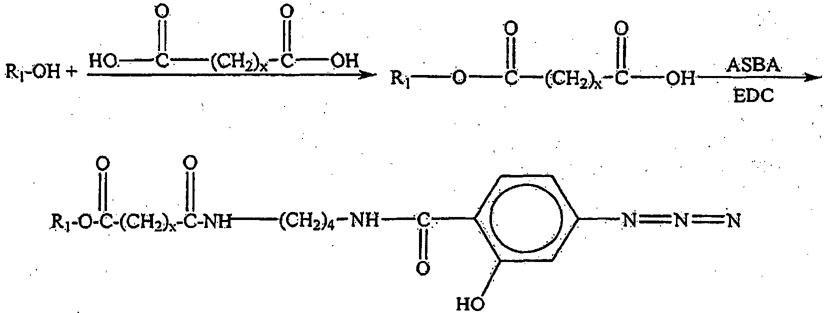 wherein R₁ is as defined above and x may be from about 1 to about 20.

The resulting polymer may then be subjected to polymerization, such as UV polymerization, to produce a composition of the present disclosure that does not possess any functionality on its ends.

In other embodiments, a reaction scheme similar to the above may be utilized to form a polymeric component of the following formulas:

R₁-[A]ᵥ-R₂ (III)

or

R₂-[A]ᵥ-R₁-[A]ᵥ-R₂ (IV)

wherein R₁ is the hydroxamate segment, R₂ is the polymer segment, i.e., an oligomer, macromer or polymer as described above, A is a bioabsorbable group as described above, and v is a number from about 1 to about 20, in embodiments from about 2 to about 6. In embodiments, R₁ may be a hydroxamate, R₂ may be a polyalkylene oxide such as a polyethylene glycol or a polyethylene glycol/polypropylene glycol copolymer, and A may be lactide, glycolide, ε-caprolactone, trimethylene carbonate, p-dioxanone, combinations thereof, and the like, as well as a diacid such as malonic acid, succinic acid, glutaric acid, adipic acid, sebacic acid, azelaic acid, combinations thereof, and the like.

Other conditions for conducting polymerization of the hydroxamates with the other components are within the purview of those skilled in the art. Polymerization may, in embodiments, be initiated by subjecting the monomers to energy including irradiation, such as high energy radiation including gamma and/or e-beam, UV light, pulse laser ablation deposition, plasma energy treatment, chemical initiation, photoinitiation, and the like. In embodiments, the use of high energy radiation initiation may be beneficial as it should not require the use of an additional initiator such as a chemical initiator or catalyst.

In other embodiments, terminal hydroxamate or polymer segments may be functionalized with bioreactive groups targeted for binding and/or bonding to tissue to function as adhesives, either by covalent, ionic, hydrogen, electrostatic, combinations thereof, and the like. For example, in embodiments, a terminal hydroxamate segment or a terminal polymer segment may be functionalized with a group capable of reacting with amines native to tissue, thereby bonding the composition of the present disclosure to the tissue to which it is applied. Suitable groups for such bonding include, but are not limited to, isocyanates, ketones, aldehydes, succinimides, epoxides, carboxylic acids, combinations thereof, and the like.

For example, the polymeric component with hydroxamate segments and polymer segments can be endcapped with groups such as isocyanates, suc cinimides, aldehydes, and combinations thereof. As used herein, succinimides also include sulfosuccinimides, succinimide esters and sulfosuccinimide esters, including N-hydroxysuccinimide ("NHS"), N-hydroxysulfosuccinimide ("SNHS"), N-hydroxyethoxylated succinimide ("ENHS"), N-hydroxysuccinimide acrylate, succinimidyl glutarate, n-hydroxysuccinimide hydroxybutyrate, combinations thereof, and the like. In embodiments, the functional group utilized for endcapping may be any functional group as described in U.S. Patent Nos. 6,566,406; 6,818,018; 7,009,034; 7,025,990; 7,211,651; and/or 7,332,566, and may be combined with the other components of the polymeric component utilizing any method within the purview of those skilled in the art, including those disclosed in U.S. Patent Nos. 6,566,406; 6,818,018; 7,009,034; 7,025,990; 7,211,651; and/or 7,332,566.

In other embodiments, for example, an isocyanate group (NCO) may be reacted with the hydroxamate segment or polymer segment so that the terminal end of the polymeric component possesses isocyanate groups. Examples of suitable isocyanates for endcapping the polymeric component include, but are not limited to, aromatic, aliphatic and alicyclic isocyanates. Examples include, but are not limited to, aromatic diisocyanates such as 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenylisocyanate), or tetramethylxylylene diisocyanate; aliphatic diisocyanates such as tetramethylene diisocyanate, hexamethylene diisocyanate, lysine diisocyanate, 2-methylpentane-1,5-diisocyanate, 3-methylpentane-1,5-diisocyanate,or 2,2,4-trimethylhexamethylene diisocyanate; and alicyclic diisocyanates such as isophorone diisocyanate, cyclohexane diisocyanate, hydrogenated xylylene diisocyanate, hydrogenated diphenylmethane diisocyanate, hydrogenated trimethylxylylene diisocyanate, 2,4,6-trimethyl 1,3-phenylene diisocyanate or commercially available DESMODURS^{®} from Bayer Material Science. Combinations of the foregoing may be utilized in embodiments. In embodiments, an aliphatic diisocyanate such as hexamethylene diisocyanate can be used.

The resulting endcapped bioabsorbable polymeric component can be linear or can have a branched or star configuration. The molecular weight of the polymeric component can be from about 100 daltons to about 20,000 daltons, in embodiments from about 300 daltons to about 10,000 daltons, in other embodiments from about 500 daltons to about 5000 daltons.

In some embodiments, the endcapped polymeric component with hydroxamate segments can be of the formulas:

R₁-[A]ᵥ-R₂-R₃ (V)

or

R₃-R₂-[A]ᵥ-R₁-[A]ᵥ-R₂-R₃ (VI)

wherein R₁ is the hydroxamate segment, R₂ is a polymer segment including an oligomer, macromer or polymer as described above, A is a bioabsorbable group, v is a number from about 1 to about 20, in embodiments from about 2 to about 6, and R₃ is a functional group utilized to endcap the polymer, including an isocyanate, in embodiments a diisocyanate as described above, as well as succinimides, sulfosuccinimides, succinimide esters and sulfosuccinimide esters, including N-hydroxysuccinimide ("NHS"), N-hydroxysulfosuccinimide ("SNHS"), N-hydroxyethoxylated succinimide ("ENHS"), N-hydroxysuccinimide acrylate, succinimidyl glutarate, n-hydroxysuccinimide hydroxybutyrate, aldehydes, combinations thereof, and the like.

The bioabsorbable groups can be present in the polymeric component so that they are present in amounts from about 5% to about 50% by weight of the final macromer composition of the present disclosure, in embodiments from about 10% to about 40% by weight of the macromer composition of the present disclosure, in other embodiments from about 15% to about 30% by weight of the macromer composition ofthe present disclosure.

A polymeric component of the present disclosure, including the above polymer segments and hydroxamate segments, may possess the hydroxamate segments in an amount of from about 1 percent by weight of the polymeric component to about 30 percent by weight of the polymeric component, in embodiments from about 5 percent by weight of the polymeric component to about 20 percent by weight of the polymeric component, with the polymer segments present in an amount of from about 5 percent by weight of the polymeric component to about 15 percent by weight ofthe polymeric component.

In addition to bioabsorbable groups, at least one linkage that is enzymatically degradable may be incorporated into the polymeric component. Linkages which are enzymatically degradable include, but are not limited to: an amino acid residue such as -Arg-, - Ala-, -Ala(D)-, -Val-, -Leu-, -Lys-, -Pro-, -Phe-, -Tyr-, -Glu-, and the like; 2-mer to 6-mer oligopeptides such as -Ile-Glu-Gly-Arg-, -Ala-Gly-Pro-Arg-, -Arg-Val-(Arg)₂-, -Val-Pro-Arg-, -Gln-Ala-Arg-, -Gln-Gly-Arg-, -Asp-Pro-Arg-, -Gln(Arg)₂ -, -Phe-Arg-, -(Ala)₃-, -(Ala)₂-, -Ala-Ala(D)-, -(Ala)₂-Pro-Val-, -(Val)₂-, -(Ala)₂-Leu-, -Gly-Leu-, -Phe-Leu-, -Val-Leu-Lys-, -Gly-Pro-Leu-Gly-Pro-, -(Ala)₂-Phe-, - (Ala)₂-Tyr-, -(Ala)₂-His-, -(Ala)₂-Pro-Phe-, -Ala-Gly-Phe-, -Asp-Glu-, -(Glu)₂ -, -Ala-Glu-, -Ile-Glu-, -Gly-Phe-Leu-Gly-, -(Arg)₂-; D-glucose, N-acetylgalactosamine, N-acetylneuraminic acid, N-acetylglucosamine, N-acetylmannnosamine or the oligosaccharides thereof; oligodeoxyribonucleic acids such as oligodeoxyadenine, oligodeoxyguanine, oligodeoxycytosine, and oligodeoxythymidine; and oligoribonucleic acids such as oligoadenine, oligoguanine, oligocytosine, oligouridine, and the like. Those skilled in the art will readily envision reaction schemes for incorporating enzymatically degradable linkages into the polymeric component.

The polymeric component possessing hydroxamate segments and polymer segments of the present disclosure can be utilized by itself or, in embodiments, combined with a second component to form a bioabsorbable macromer composition of the present disclosure. The resulting macromer composition of the present disclosure may be useful as an adhesive or sealant. For example, where the polymeric component possessing a hydroxamate segment has been endcapped with isocyanate groups, the second component of the present disclosure can possess at least one isocyanate-reactive group. In embodiments, suitable isocyanate-reactive groups may be at least one hydroxy group, at least one amine group, at least one sulfhydryl group, combinations thereof, and the like. Similarly, where the polymeric component has been functionalized with succinimides, sulfosuccinimides, n-hydroxysuccinimides, n-hydroxysulfosuccinimides, esters thereof (sometimes referred to herein as "succinimide-like groups") and the like, the second component may have groups reactive with the succinimide-like groups, including amines.

In embodiments, the second component may be selected so that it may be functionalized with appropriate groups for reacting with the polymeric component. Suitable components for use as the second component may possess at least one hydroxy groups, at least one amine group, at least one sulfhydryl group, combinations thereof, and the like.

Suitable compounds possessing at least one hydroxy group which may be utilized as the second component include water and polyols such as polyether-based polyols, polycaprolactone-based polyols, and polyhydric alcohols such as glycerol, trimethylol propane, hexane-1,2,6-triol, pentaerythritol, glucose, mannitol, disaccharides such as sucrose, sorbitol and diethylene glycol.

Suitable components possessing at least one amine group which may be utilized as the second component are within the purview of those skilled in the art and include, for example, primary amines such as bis(3-aminopropyl)amine, spermine, polyetheramine (including JEFFAMINE^{®} polyetheramines), and trilysine; as well as low molecular weight diamines, such as ethylenediamine, N-ethylethylenediamine and N,N'-diethylethylenediamine, butane-1,4-diamine, pentane-1,5-diamine, hexane-1,6-diamine, phenylene diamine; combinations thereof, and the like. In other embodiments, alkanolamines may be utilized. Examples of suitable alkanolamines include dihydric and trihydric alkanolamines, such as ethanolamine and N-ethylethanolamine. Other amines which may be utilized include triethylenediamine; N-methylmorpholine; pentamethyl diethylenetriamine; dimethylcyclohexylamine; tetramethylethylenediamine; 1-methyl-4-dimethylaminoethylpiperazine; 3-methoxy-N-dimethyl-propylamine; N-ethylmorpholine; diethylethanolamine; N-cocomorpholine; N,N-dimethyl-N',N'-dimethylisopropyl-propylene diamine; N,N-diethyl-3-diethyl aminopropylamine; and dimethyl-benzyl amine. Polymeric amines which may be utilized as the second component include polylysine; polyarginine; albumin; polyallylamine; MPC-co-acrylamide; MPC-co-polyallylamine; combinations thereof, and the like.

In embodiments, the amine utilized as the second component may be a diamine of the formula:

NH₂-R₄-NH₂ (VII)

wherein R₄ may be a polymer including any polymer segment described above, including polysaccharides, polyols, combinations thereof, and the like. In embodiments, R₄ may be a polyalkylene oxide such as polyethylene glycol, polypropylene glycol, copolymers of polyethylene glycol and polypropylene glycol, optionally containing any bioabsorbable groups as described above. Combinations of any of the foregoing amines may be utilized in embodiments.

Suitable compounds possessing at least one sulfhydryl group which may be used as the second component in forming a macromer composition of the present disclosure include, but are not limited to, thiolated gelatin, thiolated collagen, PEG-thiols, pentaerythritol tetrakis (2-mercaptoacetate), pentaerythritol tetrakis (3-mercaptopropionate), trimethylolpropane tris(2-mercaptoacetate), trimethylolpropane tris(2-mercaptopropionate), combinations thereof, and the like.

In embodiments, the polymeric component possessing a hydroxamate segment, the second component, or both, may be in a dilute solution. Suitable solvents which may be utilized to form a dilute solution include any biocompatible solvents within the purview of those skilled in the art which will not interfere in the reaction of the reactive groups of the polymeric component with the second component, for example the isocyanate-reactive groups of the second component with the isocyanate-functional groups of the polymeric component. Suitable solvents which may be utilized include, for example, polar solvents such as water, ethanol, triethylene glycol, dimethyl sulfoxide (DMSO), glymes (such as diglyme, triglyme, tetraglyme, and the like), polyethylene glycols, methoxy-polyethylene glycols, dimethylformamide, dimethylacetamide, gamma-butyrolactone, N-methylpyrollidone (NMP), ketones such as methyl ethyl ketone, cyclohexanone, diethylene glycol monoethyl ether acetate, diethylene glycol monobutyl ether acetate, diethylene glycol monomethyl ether, diethylene glycol monoethyl ether, diethylene glycol monobutyl ether, diethylene glycol monoisobutyl ether, diisobutyl ketone, diacetone alcohol, ethyl amyl ketone, ethyl lactate, combinations thereof, and the like. In other embodiments, solvents such as tetrahydrofuran, ethyl acetate, isopropyl acetate, butyl acetate, isopropanol, butanol, acetone, and the like, may be utilized. In embodiments, combinations of any ofthe foregoing solvents may be utilized to form a dilute solution.

A solvent may be mixed with the polymeric component, the second component, or both. A solvent may be mixed with the polymeric component so that the polymeric component is at a concentration of from about 1 weight percent to about 90 weight percent of the first solution, in embodiments from about 5 weight percent to about 40 weight percent of the first solution. A solvent may be mixed with the second component so that the second component is at a concentration of from about 1 weight percent to about 90 weight percent of the second solution, in embodiments from about 5 weight percent to about 40 weight percent of the second solution.

The amount of solvent used will depend on a number of factors, including the particular polymeric component, second component, or both, that are to be employed and the intended end use of the composition.

The mixture of either the polymeric component or second component and solvent as described herein may result in an emulsion or a diluted solution. The viscosity of the resulting emulsion or solution may be from about 100 cP to about 100,000 cP, in other embodiments from about 1,000 cP to about 80,00 cP, in still other embodiments from about 5,000 cP to about 20,000 cP.

In embodiments, the second component may be mixed with the polymeric component at a ratio of from about 1:10 to about 10:1 by weight, in embodiments, at a ratio of from about 5:1 to about 1:1 by weight.

Where utilized, the second component may be present in the final composition of the present disclosure in an amount of from about 5% to about 90% by weight of the macromer composition, in embodiments from about 10% to about 80% by weight of the macromer composition, in other embodiments from about 15% to about 50% by weight of the macromer composition. The polymeric component may thus be present in an amount of from about 10% to about 95% by weight of the macromer composition, in embodiments from about 20% to about 90% by weight of the macromer composition, in other embodiments from about 50% to about 85% by weight of the macromer composition.

The concentrations of the polymeric component and the second component will vary depending upon a number of factors, including the types and molecular weights of the particular polymers used and the desired end use application, i.e., as an adhesive or sealant.

Where utilized alone, the polymeric component with appropriate functional groups, in embodiments possessing a hydroxamate segment, can react or cross-link in situ to form a biocompatible adhesive or sealant (e.g., an isocyanate (NCO) group reacting with water and/or tissue; or, an n-hydroxy succinimide (NHS) group reacting with tissue; etc.). Where combined with the second component described above, the two components cross-link in situ when mixed together to form a biocompatible macromer composition suitable for use as an adhesive or sealant. The polymeric component possessing a hyroxamate segment, optionally in combination with the second component, rapidly forms a three dimensional gel-like adhesive matrix, which reduces total surgical/operating time during a medical procedure.

Where the polymeric component is used alone to form the bioabsorbable macromer composition of the present disclosure, the polymeric component possessing a hydroxamate segment and optional isocyanate groups can be exposed to water, optionally in the presence of a catalyst, to form a bioabsorbable macromer composition of the present disclosure. In embodiments, foaming agents may be added, for example carbonates including sodium bicarbonate, optionally in combination with an organic acid such as citric acid. In other embodiments, initiators may be included.

In other embodiments, the bioabsorbable macromer composition may be prepared by combining the polymeric component with the second component to form a three-dimensional crosslinked matrix. Cross-linking may be performed by exposing the components to water in the presence or absence of a catalyst, such as a tertiary amine catalyst. Suitable catalysts for use in the cross-linking reaction include 1,4-diazobicyclo [2.2.2] octane, triethylamine, diethylaminoethanol, dimethlyamino pyridine, stannous octoate, etc. The amount of catalyst employed can be from about 0.5 grams to about 50 grams per kilogram of the components being cross-linked, in embodiments from about 1 gram to about 10 grams per kilogram of the components being cross-linked.

The exact reaction conditions for achieving cross-linking of the polymeric component, optionally in combination with the second component, can vary depending on a number of factors such as the composition of the polymer, the degree of endcapping with additional functional groups such as isocyanates, the specific isocyanate utilized, and the desired degree of cross-linking. The cross-linking reaction may be conducted at temperatures of from about 20°C to about 40°C, in embodiments from about 25°C to about 35°C, for a period of time from about 5 minutes to about 72 hours or more, in embodiments from about 1 hour to about 36 hours.

For the bioabsorbable macromer composition of the present disclosure, the use of higher concentrations of the polymeric component and optional second component may result in the formation of a more tightly crosslinked bioabsorbable macromer composition, producing a stiffer and stronger gel matrix. As such, bioabsorbable macromer compositions of the present disclosure intended for use in tissue augmentation may use higher concentrations of the polymeric and optional second components. Bioabsorbable macromer compositions of the present disclosure intended for use as bioadhesives or for the prevention of post-surgical adhesions need not be as firm and may therefore contain lower concentrations of the components.

Biologically active agents may be included in the bioabsorbable macromer compositions of the present disclosure. For example, naturally occurring polymers, including proteins such as collagen and derivatives of various naturally occurring polysaccharides such as glycosaminoglycans, can be utilized in forming the polymeric component or incorporated into the bioabsorbable macromer compositions of the present disclosure. When these other biologically active agents also contain functional groups, the groups may react with functional groups on the polymeric and/or optional second components of the bioabsorbable macromer compositions of the present disclosure.

A variety of optional ingredients including medicinal agents may also be added to the bioabsorbable macromer compositions of the present disclosure. Medicinal agents which may be added include antimicrobial agents, colorants, preservatives, or medicinal agents such as, for example, protein and peptide preparations, antipyretic, antiphlogistic and analgesic agents, anti-inflammatory agents, vasodilators, antihypertensive and antiarrhythmic agents, hypotensive agents, antitussive agents, antineoplastics, local anesthetics, hormone preparations, antiasthmatic and antiallergic agents, antihistaminics, anticoagulants, antispasmodics, cerebral circulation and metabolism improvers, antidepressant and antianxiety agents, vitamin D preparations, hypoglycemic agents, antiulcer agents, hypnotics, antibiotics, antifungal agents, sedative agents, bronchodilator agents, antiviral agents and dysuric agents.

Where the bioabsorbable macromer composition is intended for delivery of a drug or protein, the amounts of the polymeric component possessing a hydroxamate segment and optional second components can be adjusted to promote the initial retention of the drug or polymer in the bioabsorbable macromer composition and its subsequent release. Methods and means for making such adjustments will be readily apparent to those skilled in the art.

Imaging agents such as iodine or barium sulfate, or fluorine, can also be combined with the bioabsorbable macromer compositions of the present disclosure to allow visualization of the surgical area through the use of imaging equipment, including X-ray, MRI, and CAT scan.

Additionally, an enzyme may be added to the bioabsorbable macromer compositions of the present disclosure to increase their rate of degradation. Suitable enzymes include, for example, peptide hydrolases such as elastase, cathepsin G, cathepsin E, cathepsin B, cathepsin H, cathepsin L, trypsin, pepsin, chymotrypsin, γ-glutamyltransferase (γ-GTP) and the like; sugar chain hydrolases such as phosphorylase, neuraminidase, dextranase, amylase, lysozyme, oligosaccharase and the like; oligonucleotide hydrolases such as alkaline phosphatase, endoribonuclease, endodeoxyribonuclease and the like. In some embodiments, where an enzyme is added, the enzyme may be included in a liposome or microsphere to control the rate of its release, thereby controlling the rate of degradation of the bioabsorbable macromer composition of the present disclosure. Methods for incorporating enzymes into liposomes and/or microspheres are with the purview ofthose skilled in the art.

The resulting bioabsorbable, macromer compositions can be used in a medical/surgical capacity in place of, or in combination with, sutures, staples, clamps, meshes, soft tissue repair devices, grafts, valves, pins, rods, anchors, and the like. In embodiments, the bioabsorbable macromer compositions can be used to seal or adhere delicate tissue together, such as lung tissue, in place of conventional tools that may cause mechanical stress. The resulting bioabsorbable macromer compositions can also be used to seal air and/or fluid leaks in tissue as well as to prevent post-surgical adhesions and to fill voids and/or defects in tissue.

The bioabsorbable macromer compositions of the present disclosure can also act as drug carriers, allowing controlled release and direct delivery of a drug to a specific location in an animal, especially a human.

The bioabsorbable macromer compositions of the present disclosure can be used for a number of different human and animal medical applications including, but not limited to, wound closure (including surgical incisions and other wounds), adhesives for adhering medical devices (including implants) to tissue, sealants and void fillers, and embolic agents. Adhesives may be used to bind tissue together either as a replacement of, or as a supplement to, sutures, staples, tapes and/or bandages. Use of the disclosed bioabsorbable macromer composition can eliminate or substantially reduce the number of sutures normally required during current practices, and eliminate the subsequent need for removal of staples and certain types of sutures. The disclosed bioabsorbable macromer composition can thus be particularly suitable for use with delicate tissues where sutures, clamps or other conventional tissue closure mechanisms may cause further tissue damage.

Additional applications include use of the bioabsorbable macromer compositions as sealants for sealing tissues to prevent or control blood or other fluid leaks at suture or staple lines. In another embodiment, the bioabsorbable macromer compositions can be used to attach skin grafts and position tissue flaps during reconstructive surgery. In still another embodiment, the bioabsorbable macromer compositions can be used to close tissue flaps in periodontal surgery.

In other embodiments, especially where the bioabsorbable macromer composition of the present disclosure is to be utilized as an implant or a void filler or sealant to fill a defect in an animal's body, it may be advantageous to more precisely control the conditions and extent of cross-linking; thus, it may be desirable to partially cross-link the macromer composition prior to its use to fill a void in animal tissue. In such a case the bioabsorbable macromer composition of the present disclosure can be applied to the void or defect and allowed to set, thereby filling the void or defect.

To effectuate the joining of two tissue edges, the two edges are approximated, and the polymeric component, i.e., the polymeric component possessing a hydroxamate segment, optionally endcapped with a functional group such as an isocyanate, may be applied alone or in combination with the optional second component. The component(s) crosslink rapidly, generally taking less than one minute. It is believed that a functional group such as an isocyanate groups of the polymeric component possessing a hydroxamate segment may adhere to tissue by linking directly to amine groups present on the tissue surface. In this case the macromer composition of the present disclosure can be used as an adhesive to close a wound, including a surgical incision. The macromer composition of the present disclosure can thus be applied to the wound and allowed to set, thereby closing the wound.

The present disclosure is also directed to a method for using the bioabsorbable macromer composition of the present disclosure to adhere a medical device to tissue. In embodiments, depending on the composition of the medical device, a coating may be required on the medical device. In some cases such a coating can include the polymeric component of the bioabsorbable macromer composition of the present disclosure, or where utilized, the second component. In some aspects, the medical device includes an implant. Other medical devices include, but are not limited to, pacemakers, stents, shunts, and the like. Generally, for adhering a device to the surface of animal tissue, the polymeric component, second component and/or macromer composition of the present disclosure can be applied to the device, the tissue surface, or both. The device and tissue surface are then brought into contact with each other and the bioabsorbable macromer composition is allowed to form/set, thereby adhering the device and surface to each other.

In some embodiments the polymeric component could be applied to tissue, the second component applied to a device, or vice-versa, and the two contacted with each other to form a bioabsorbable macromer composition adhering the device to tissue.

The present bioabsorbable macromer composition can also be used to prevent post surgical adhesions. In such an application, the bioabsorbable macromer composition is applied and cured as a layer on surfaces of internal tissues in order to prevent the formation of adhesions at a surgical site during the healing process. In addition to the formation of adhesion barriers, the composition of the present disclosure may be utilized to form implants such as gaskets, buttresses, or pledgets for implantation.

When used as a sealant, the bioabsorbable macromer composition of the present disclosure can be used in surgery to prevent or inhibit bleeding or fluid leakage both during and after a surgical procedure. It can also be applied to prevent air leaks associated with pulmonary surgery. The macromer composition may be applied directly to the desired area in at least an amount necessary to seal off any defect in the tissue and seal off any fluid or air movement.

Application of the bioabsorbable macromer composition, whether as an adhesive or sealant, with or without other additives, can be done by any conventional means. These include dripping, brushing, or other direct manipulation of the bioabsorbable macromer composition on the tissue surface, or spraying of the bioabsorbable macromer composition onto the surface. In open surgery, application by hand, forceps or the like, is contemplated. In endoscopic surgery, the bioabsorbable macromer composition can be delivered through the cannula of a trocar, and spread at the site by any device within the purview of those skilled in the art.

In embodiments, the bioabsorbable macromer composition can be dispensed from a conventional adhesive dispenser, which can provide mixing of the polymeric and optional second components prior to the dispenser. Such dispensers are disclosed, for example, in U.S. Patent Nos. 4,978,336; 4,361,055; 4,979,942; 4,359,049; 4,874,368; 5,368,563; and 6,527,749.

The present bioabsorbable macromer compositions have various advantageous properties. The bioabsorbable macromer compositions of the present disclosure are safe, possess enhanced adherence to tissue, are biodegradable, have enhanced hemostatic potential, have low cost, and are easy to prepare and use. By varying the selection of the components, the strength and elasticity of the bioabsorbable macromer composition can be controlled, as can the gelation time.

The bioabsorbable macromer compositions rapidly form a compliant gel matrix, which insures stationary positioning of tissue edges or implanted medical devices in the desired location and lowers overall required surgical/application time. The bioabsorbable macromer compositions form strong cohesive bonds. They exhibit excellent mechanical performance and strength, while retaining the necessary pliability to adhere living tissue. This strength and pliability allows a degree of movement of tissue without shifting the surgical tissue edge. Additionally, the bioabsorbable macromer compositions are biodegradable, allowing the degradation components to pass safely through the subject's body.

## Claims

1. A bioabsorbable macromer composition of the formula selected from the group consisting of
R₁-[A]ᵥ-R₂-R₃
and
R₃-R₂-[A]ᵥ-R₁-[A]ᵥ-R₂-R₃
wherein R₁ comprises a hydroxamate segment, R₂ comprises a polymer selected from the group consisting of polysaccharides and polyols, R₃ comprises a functional group selected from the group consisting of isocyanates, succinimides, aldehydes, and combinations thereof, A is a bioabsorbable group, and v is a number from 1 to 20.

2. A bioabsorbable macromer composition as in claim 1, wherein R₂ comprises a polyol selected from the group consisting of polyethylene oxide, polyethylene glycol, polypropylene glycol, polyethylene oxide - polypropylene oxide copolymers, polyethylene glycol-adipate, polyethylene glycol-polypropylene glycol copolymers, and combinations thereof.

3. A bioabsorbable macromer composition as in claim 2, wherein R₂ comprises polyethylene glycol.

4. A bioabsorbable macromer composition as in claim 1, wherein R₂ comprises a polysaccharide selected from the group consisting of sorbitol, mannitol, sucrose, dextran, cyclodextrin, and combinations thereof.

5. A bioabsorbable macromer composition as in any preceding claim, wherein the bioabsorbable group is selected from the group consisting of lactic acid, glycolic acid, 1,4-dioxane-2-one, 1,3-dioxane-2-one, succinnic acid, adipic acid, sebacic acid, malonic acid, glutaric acid, azelaic acid, ethyl dichlorophosphate, sebacic acid anhydride, azelaic acid anhydride, and combinations thereof.

6. A bioabsorbable macromer composition as in any preceding claim, wherein v is a number from 2 to 6.

7. A bioabsorbable macromer composition as in any preceding claim, wherein the hydroxamate segment comprises at least one hydroxamate of the formula 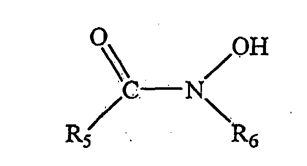 wherein R₅ is selected from the group consisting of vinyl groups, hydroxyl alkyl acrylate groups, hydoxy alkyl methacrylate groups, alkyl groups, alkoxy groups, alkenyl groups, acrylamides, methacrylamides, polymers, and combinations thereof, and R₆ is selected from the group consisting of hydrogen, alkyl groups, alkoxy groups, alkenyl groups, and combinations thereof.

8. A bioabsorbable macromer composition as in any preceding claim, wherein the isocyanate is selected from the group consisting of 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenyl isocyanate), tetramethylxylylene diisocyanate, tetramethylene diisocyanate, hexamethylene diisocyanate, lysine diisocyanate, 2-methylpentane-1,5-diisocyanate, 3-methylpentane-1,5-diisocyanate, 2,2,4-trimethylhexamethylene diisocyanate, isophorone diisocyanate, cyclohexane diisocyanate, hydrogenated xylylene diisocyanate, hydrogenated diphenylmethane diisocyanate, hydrogenated trimethylxylylene diisocyanate, 2,4,6-trimethyl 1,3-phenylene diisocyanate, and combinations thereof.

9. A bioabsorbable macromer composition as in any preceding claim for use in closing a wound wherein the method comprises the step of applying the composition to the wound; and
allowing the bioabsorbable macromer composition to set thereby closing the wound.

10. The bioabsorbable macromer composition of claim 9 wherein the wound is a surgical incision.

11. A bioabsorbable macromer composition as in any preceding claim for use in filling a void in animal tissue wherein the method comprises the steps of applying the composition to the void; and
allowing the bioabsorbable macromer composition to set thereby filling the void.

## Patentansprüche

1. Biologisch absorbierbare Makromerzusammensetzung der Formel, ausgewählt aus der Gruppe, bestehend aus
R₁-[A]ᵥ-R₂-R₃
und
R₃-R₂-[A]ᵥ-R₁-[A]ᵥ-R₂-R₃
wobei R₁ ein Hydroxamatsegment beinhaltet, R₂ ein Polymer, ausgewählt aus der Gruppe, bestehend aus Polysacchariden und Polyolen, beinhaltet, R₃ eine funktionelle Gruppe, ausgewählt aus der Gruppe, bestehend aus Isocyanaten, Succinimiden, Aldehyden und Kombinationen davon, beinhaltet, A eine biologisch absorbierbare Gruppe ist und v eine Zahl von 1 bis 20 ist.

2. Biologisch absorbierbare Makromerzusammensetzung gemäß Anspruch 1, wobei R₂ ein Polyol, ausgewählt aus der Gruppe, bestehend aus Polyethylenoxid, Polyethylenglycol, Polypropylenglycol, Polyethylenoxid-Polypropylenoxid-Copolymeren, Polyethylenglycol-Adipat, Polyethylenglycol-Polypropylenglycol-Copolymeren und Kombinationen davon, beinhaltet.

3. Biologisch absorbierbare Makromerzusammensetzung gemäß Anspruch 2, wobei R₂ Polyethylenglycol beinhaltet.

4. Biologisch absorbierbare Makromerzusammensetzung gemäß Anspruch 1, wobei R₂ ein Polysaccharid, ausgewählt aus der Gruppe, bestehend aus Sorbitol, Mannitol, Saccharose, Dextran, Cyclodextrin und Kombinationen davon, beinhaltet.

5. Biologisch absorbierbare Makromerzusammensetzung gemäß einem vorhergehenden Anspruch, wobei die biologisch absorbierbare Gruppe aus der Gruppe, bestehend aus Milchsäure, Glycolsäure, 1,4-Dioxan-2-on, 1,3-Dioxan-2-on, Bernsteinsäure, Adipinsäure, Sebacinsäure, Malonsäure, Glutarsäure, Azelainsäure, Ethyldichlorphosphat, Sebacinsäureanhydrid, Azelainsäureanhydrid und Kombinationen davon, ausgewählt ist.

6. Biologisch absorbierbare Makromerzusammensetzung gemäß einem vorhergehenden Anspruch, wobei v eine Zahl von 2 bis 6 ist.

7. Biologisch absorbierbare Makromerzusammensetzung gemäß einem vorhergehenden Anspruch, wobei das Hydroxamatsegment mindestens ein Hydroxamat der folgenden Formel beinhaltet: 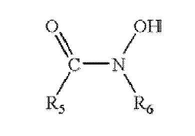 wobei R₅ aus der Gruppe, bestehend aus Vinylgruppen, Hydroxylalkylacrylatgruppen, Hydroxyalkylmethacrylatgruppen, Alkylgruppen, Alkoxygruppen, Alkenylgruppen, Acrylamiden, Methacrylamiden, Polymeren und Kombinationen davon, ausgewählt ist, und R₆ aus der Gruppe, bestehend aus Wasserstoff, Alkylgruppen, Alkoxygruppen, Alkenylgruppen und Kombinationen davon, ausgewählt ist.

8. Biologisch absorbierbare Makromerzusammensetzung gemäß einem vorhergehenden Anspruch, wobei das Isocyanat aus der Gruppe ausgewählt ist, die aus Folgenden besteht: 2,4-Toluendiisocyanat, 2,6-Toluendiisocyanat, 2,2'-Diphenylmethandiisocyanat, 2,4'-Diphenylmethandiisocyanat, 4,4'-Diphenylmethandiisocyanat, Diphenyldimethylmethandiisocyanat, Dibenzyldiisocyanat, Naphthylendiisocyanat, Phenylendiisocyanat, Xylylendiisocyanat, 4,4'-Oxybis(phenylisocyanat), Tetramethylxylylendiisocyanat, Tetramethylendiisocyanat, Hexamethylendiisocyanat, Lysindiisocyanat, 2-Methylpentan-1,5-diisocyanat, 3-Methylpentan-1,5-diisocyanat, 2,2,4-Trimethylhexamethylendiisocyanat, Isophorondiisocyanat, Cyclohexandiisocyanat, hydriertes Xylylendiisocyanat, hydriertes Diphenylmethandiisocyanat, hydriertes Trimethylxylylendiisocyanat, 2,4,6-Trimethyl-1,3-phenylendiisocyanat und Kombinationen davon.

9. Biologisch absorbierbare Makromerzusammensetzung gemäß einem vorhergehenden Anspruch zur Verwendung beim Verschließen einer Wunde, wobei das Verfahren Folgendes beinhaltet: den Schritt des Applizierens der Zusammensetzung auf die Wunde; und
das Festwerdenlassen der biologisch absorbierbaren Makromerzusammensetzung, um somit die Wunde zu verschließen.

10. Biologisch absorbierbare Makromerzusammensetzung gemäß Anspruch 9, wobei die Wunde eine chirurgische Inzision ist.

11. Biologisch absorbierbare Makromerzusammensetzung gemäß einem vorhergehenden Anspruch zur Verwendung beim Füllen eines Hohlraums in tierischem Gewebe, wobei das Verfahren die folgenden Schritte beinhaltet: das Applizieren der Zusammensetzung in den Hohlraum; und
das Festwerdenlassen der biologisch absorbierbaren Makromerzusammensetzung, um somit den Hohlraum zu füllen.

## Revendications

1. Composition de macromère bioabsorbable de formule choisie dans le groupe constitué des suivantes :
**R₁-[A]ᵥ-R₂-R₃**
et
**R₃-R₂-[A]ᵥ-R₁-[A]ᵥ-R₂-R₃**
dans lesquelles R₁ comprend un segment d'hydroxamate, R₂ comprend un polymère choisi dans le groupe constitué des polysaccharides et des polyols, R₃ comprend un groupement fonctionnel choisi dans le groupe constitué des isocyanates, des succinimides, des aldéhydes et de leurs combinaisons, A est un groupement bioabsorbable et v est un nombre de 1 à 20.

2. Composition de macromère bioabsorbable selon la revendication 1, dans laquelle R₂ comprend un polyol choisi dans le groupe constitué du poly(oxyde d'éthylène), du polyéthylèneglycol, du polypropylèneglycol, des copolymères de poly(oxyde d'éthylène) et de poly(oxyde de propylène), du polyéthylèneglycol-adipate, des copolymères de polyéthylèneglycol et de polypropylèneglycol et de leurs combinaisons.

3. Composition de macromère bioabsorbable selon la revendication 2, dans laquelle R₂ comprend du polyéthylèneglycol.

4. Composition de macromère bioabsorbable selon la revendication 1, dans laquelle R₂ comprend un polysaccharide choisi dans le groupe constitué du sorbitol, du mannitol, du saccharose, du dextrane, de la cyclodextrine et de leurs combinaisons.

5. Composition de macromère bioabsorbable selon l'une quelconque des revendications précédentes, dans laquelle le groupement bioabsorbable est choisi dans le groupe de l'acide lactique, de l'acide glycolique, de la 1,4-dioxan-2-one, de la 1,3-dioxan-2-one, de l'acide succinique, de l'acide adipique, de l'acide sébacique, de l'acide malonique, de l'acide glutarique, de l'acide azélaïque, du dichlorophosphate d'éthyle, de l'anhydride d'acide sébacique, de l'anhydride d'acide azélaïque et de leurs combinaisons.

6. Composition de macromère bioabsorbable selon l'une quelconque des revendications précédentes, dans laquelle v est un nombre de 2 à 6.

7. Composition de macromère bioabsorbable selon l'une quelconque des revendications précédentes, dans laquelle le segment d'hydroxamate comprend au moins un hydroxamate de formule : 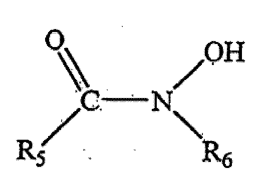 dans laquelle R₅ est choisi dans le groupe constitué des groupements vinyle, des groupements acrylate d'hydroxyalkyle, des groupements méthacrylate d'hydroxyalkyle, des groupements alkyle, des groupements alcoxy, des groupement alcényle, des acrylamides, des méthacrylamides, des polymères et de leurs combinaisons, et R₆ est choisi dans le groupe constitué de l'hydrogène, des groupements alkyle, des groupement alcoxy, des groupements alcényle et de leurs combinaisons.

8. Composition de macromère bioabsorbable selon l'une quelconque des revendications précédentes, dans laquelle l'isocyanate est choisi dans le groupe constitué du diisocyanate de 2,4-toluène, du diiisocyanate de 2,6-toluène, du diisocyanate de 2,2'-diphénylméthane, du diisocyanate de 2,4'-diphénylméthane, du diisocyanate de 4,4'-diphényl-méthane, du diisocyanate de diphényldiméthyl-méthane, du diisocyanate de dibenzyle, du diisocyanate de naphtylène, du diisocyanate de phénylène, du diisocyanate de xylylène, du 4,4'-oxybis(isocyanate de phényle), du diisocyanate de tétraméthylxylylène, du diisocyanate de tétraméthylène, du diisocyanate d'hexaméthylène, du diisocyanate de lysine, du 2-méthylpentane-1,5-diisocyanate, du 3-méthylpentane-1,5-diisocyanate, du diisocyanate de 2,2,4-triméthylhexaméthylène, du diisocyanate d'isophorone, du diisocyanate de cyclohexane, du diisocyanate de xylylène hydrogéné, du diisocyanate de diphénylméthane hydrogéné, du diisocyanate de triméthylxylylène hydrogéné, du diisocyanate de 2,4,6-triméthyl-1,3-phénylène et de leurs combinaisons.

9. Composition de macromère bioabsorbable selon l'une quelconque des revendications précédentes pour usage dans l'obturation d'une plaie, dans laquelle le procédé comprend les étapes consistant à :
appliquer la composition à la plaie ; et
permettre à la composition de macromère bioabsorbable de se figer, obturant de la sorte la plaie.

10. Composition de macromère bioabsorbable selon la revendication 9, dans laquelle la plaie est une incision chirurgicale.

11. Composition de macromère bioabsorbable selon l'une quelconque des revendications précédentes pour usage dans l'obturation d'un vide dans un tissu animal, dans laquelle le procédé comprend les étapes consistant à :
appliquer la composition au vide ; et
permettre à la composition de macromère bioabsorbable de se figer, obturant de la sorte le vide.
